# EUROPÄISCHE PATENTSCHRIFT

(11) **EP 0 615 444 B1**
(45) Veröffentlichungstag und Bekanntmachung des Hinweises auf die Patenterteilung: **06.03.1996**
(21) Anmeldenummer: 92924546.2
(22) Anmeldetag: 04.12.1992
(51) Int. Cl.: A61K 9/51, A61K 9/10, A61K 9/16

(54) **PERORALE APPLIKATIONSFORM FÜR PEPTIDARZNEISTOFFE, INSBESONDERE INSULIN**
PERORAL ADMINISTRATION FORM FOR PEPTIDIC MEDICAMENTS, IN PARTICULAR INSULIN
FORME D'ADMINISTRATION PERORALE DE MEDICAMENTS PEPTIDIQUES, NOTAMMENT L'INSULINE

(30) Priorität: 05.12.1991 DE 4140186; 05.12.1991 DE 4140195; 05.12.1991 DE 4140178; 05.12.1991 DE 4140177; 30.04.1992 US 876867
(43) Veröffentlichungstag der Anmeldung: 21.09.1994
(73) Patentinhaber: ALFATEC-PHARMA GmbH, D-69120 Heidelberg (DE)
(72) Erfinder: WUNDERLICH, Jens-Christian, D-6900 Heidelberg (DE); SCHICK, Ursula, D-69198 Schriesheim (DE); WERRY, Jürgen, D-6700 Ludwigshafen (DE); FREIDENREICH, Jürgen, D-6905 Schriesheim (DE)
(74) Vertreter: Fürniss, Peter, Dr.
(86) Internationale Anmeldenummer: DE9201009
(87) Internationale Veröffentlichungsnummer: WO9310767

(56) Entgegenhaltungen:
- EP-A- 0 138 216
- EP-A- 0 230 654
- WO-A-85/05029
- WO-A-89/03207
- DE-A- 3 106 984
- US-A- 3 312 594
- AMERICAN PHARMACEUTICAL ASSOCIATION / PHARMACEUTICAL SOCIETY OF GREAT BRITAIN 'HANDBOOK OF PHARMACEUTICAL EXCIPIENTS' 1986 , AMERICAN PHARM. ASSOC. , WASHINGTON
- DATABASE WPIL Section Ch, Week 9121, 11. April 1991 Derwent Publications Ltd., London, GB; Class A12, AN 91-152292

## Beschreibung

Die Erfindung betrifft eine perorale Applikationsform für Peptidarzneimittel, die mindestens einen Peptidarzneistoff, in einer Matrix aus Gelatine oder einem Gelatinederivat verteilt, neben pharmazeutisch üblichen Trägern und Hilfsstoffen enthält. Die Erfindung betrifft weiterhin ein Verfahren zur Herstellung einer solchen peroralen Applikationsform.

In den hochindustrialisierten Ländern kann man davon ausgehen, daß ca. 2-3 % der Bevölkerung das Krankheitsbild Diabetes zeigen. Zur effektiven Behandlung dieser Erkrankung mit ihren wichtigsten Symptomen Hyperglykämie, Polyurie, Glucosurie, sowie Hyperlipidämie, ist man heute, trotz der enormen Vielfalt pharmazeutischer Entwicklungen, nach wie vor auf die exogene Zufuhr von Insulin angewiesen. Auch die oralen Antidiabetika vom Typ der Sulfonylharnstoffe, die nur dann indiziert sind, wenn die körpereigene Insulinproduktion weigstens noch z.T. erhalten ist, bieten höchstens eine begrenzte Anwendungsbreite.

Die Applikation von Insulin geschieht weitestgehend durch Injektion (parenterale Applikation). Andere Applikationswege, z.B. die nasale, pulmonale, rektale oder vor allem die perorale Applikation befinden sich derzeit in Erprobung. Es ist jedoch noch nicht bekannt geworden, daß ein entsprechendes Präparat Marktreife erlangen konnte. Vielmehr befindet man sich noch um Stadium der orientierenden Untersuchungen. Bekanntlich sind Injektionen mit Nachteilen verbunden. So kann an der Applikationsstelle beispielsweise Lipodystrophie oder andere Fremdkörperreaktionen auftreten. Probleme mit der Handhabung von Injektionsspritzen sind besonders bei sehr jungen und älteren Patienten zu erwarten. Eine regelmäßig erforderliche Injektion muß bei diesen Patientengruppen oft durch eine betreuende Person vorgenommen werden. Es ist daher offensichtlich, daß dieser Aufwand die Patienten-Compliance nicht gerade fördert.

Die optimale, einfachste und sicherste Anwendung von Arzneistoffen stellt dagegen die perorale Applikation, beispielsweise von Tabletten, Kapseln oder Trinklösungen dar. Im Falle von Peptidarzneistoffen, wie z.B. Insulin, ergeben sich aber ausgeprägte Schwierigkeiten, weil diese nach Freisetzung im Gastrointestinaltrakt (GIT; Magen oder Dünndarm) bereits vor der Resorption durch enzymatischen Abbau zum größten Teil inaktiviert werden. Enzymatischer Abbau in der Magen- oder Dünndarmflüssigkeit oder auf der Mucosa droht die Bioverfügbarkeit von Peptidarzneistoffen, besonders Insulin, auf ein Minimum zu senken. Außerdem entfällt für Peptidarzneistoffe der Resorptionsmechanismus durch passiven Transport weitgehend. Das liegt zum einen an der Molekülgröße, denn die Ausschlußgrenze für den passiven Transport wird bei ca. 500 Dalton angenommen. Andererseits erschweren substanzspezifische Eigenschaften, wie Hydrophilie (niedriger Verteilungskoeffizient), Selbstassoziation zu größeren Einheiten oder Bindung an Bestandteile des Gastrointestinaltrakts die Resorption. Ferner wird die Resorption zusätzlich erschwert, wenn durch Dissoziation funktioneller Wirkstoffgruppen entstehende negative Ladung zu elektrostatischer Abstoßung an der Glycocalyx führt, der negativ geladenen Glykoproteinschicht, die der Lipiddoppelschicht aufliegt. Resorption von Peptidarzneistoffen ist aber trotzdem von außerordentlicher Bedeutung, wenn man eine parenterale Zufuhr erfolgreich umgehen will.

Es wurde schon vorgeschlagen, Insulin in Liposomen eingekapselt zu verabreichen. Bei diesen Untersuchungen schien es allerdings nicht möglich, die resorbierte Insulinmenge quantitativ zu bestimmen. Daher können diese Versuche wohl nur grobe Orientierungswerte bieten. Die Anwendung von Liposomen bringt darüberhinaus bekanntlich Schwierigkeiten sowohl bei der Herstellung als auch bei der Lagerung entsprechender Arzneiformen mit sich.

In jüngerer Zeit wurde über brauchbare Ansätze berichtet, um Insulin peroral applizieren zu können. Von Interesse sind dabei besonders Arzneiformen, die magen- und dünndarmresistent sind, und erst nach Erreichen des peptidasearmen Colons das Insulin freisetzen.

Es wurde ebenfalls schon vorgeschlagen, Insulin zusammen mit einem Resorptionsbeschleuniger in eine Weichgelatinekapsel einzubringen (EP Appl. 0 225 189), wobei die Kapsel mit einem Überzug versehen ist, der sich erst im Colon auflösen soll, und das Insulin zusammen mit besagtem Resorptionsbeschleuniger freisetzt. Der Einsatz von Resorptionsbeschleunigern (z.B. bestimmte Tenside bzw. Salicylsäurederivate) im GIT scheint jedoch wegen der dort erfolgenden hohen Verdünnung nur begrenzte Effektivität zu haben. Die aus diesem Grunde eingesetzte sehr große Menge, die bis zu 50 % des Kapselinhalts ausmacht, kann bereits schädliche Nebenwirkungen hervorrufen. Außerdem sind die u.U. toxischen Nebenwirkungen von Tensiden, besonders bei der Einwirkung auf Schleimhäute, hinreichend bekannt. Am Einsatz von Salicylsäurederivaten als pharmazeutisch gebräuchliche Hilfsstoffe dürfen allerdings berechtigte Zweifel angebracht werden.

US-PS 4.849.405 schlägt die Einbettung von Insulin in ein flüssiges, wäßriges Zweiphasensystem auf Koazervatbasis vor. Koazervate verhalten sich jedoch bekanntlich nicht unkritisch bei der Herstellung. Eine genaue Überwachung der Prozeßparameter ist unabdingbar. Die Reproduzierbarkeit des Verfahrens ist daher in Frage zu stellen. Das in diesem Koazervat eingebettete Insulin soll eine schnell freisetzende Arzneiform darstellen, wobei die Zubereitung in flüssiger Form (Emulsion) vorliegt. Zu der Lagerstabilität dieses Systems dürfen jedoch berechtigte Bedenken angemeldet werden. Das Koazervat kann durch Wärmebehandlung (Härtung) oder durch Vernetzung mit Aldehyden (z.B. Glutaraldehyd), anschließende Abtrennung der Mikrokapseln durch Filtration und Trocknung in eine lagerstabile, nun verzögert freisetzende Arzneiform überführt werden. Bei diesen Prozessen ist aber ein Aktivitätsverlust des Insulins durch chemische Veränderung nicht auszuschließen. Es ist bekannt, daß Insulin sowohl empfindlich gegen Hitze ist, als auch gegenüber Aldehyden sich wohl kaum inert verhalten wird. Außerdem ist generell bei dem in US-Pat. 4.849.405 angegebenen Verfahren mit einem hohen Insulinverlust bei der Einkapselung zu rechnen, was sich mit Sicherheit auf die Herstellungskosten niederschlägt. Über die Ausbeute des eingekapselten Insulins wird nichts berichtet.

Der vorliegenden Erfindung liegt nun die Aufgabe zugrunde, ein Arzneimittel für die perorale Applikation von Peptidarzneistoffen, insbesondere Insulin, bereitzustellen, das die im Stand der Technik geschilderten Probleme bei dieser Applikationsart überwindet und somit eine sichere und effektive Behandlung ermöglicht.

Diese Aufgabe wird erfindungsgemäß durch ein Arzneimittel, das Peptidarzneistoffe, insbesondere Insulin, in einer Gelatinematrix neben üblichen pharmazeutischen Trägern und Hilfsstoffen enthält, gelöst. Weiterhin wird diese Aufgabe dadurch gelöst, daß der Peptidarzneistoff, insbesondere Insulin, als geladenes Molekül durch adsorptive Ladungskompensation (Pseudokoazervat) mit einer entgegengesetzt geladenen Gelatine assoziiert ist. Schließlich wird die Aufgabe auch durch die Verwendung eines, durch adsorptive Ladungskompensation (Pseudokoazervat) an eine entgegengesetzt geladene Gelatine assoziierten Systems von Peptidarzneimittel, insbesondere Insulin, zur Herstellung von Arzneimitteln, die zur sicheren und effektiven Behandlung des Krankheitsbildes Diabetes geeignet sind, gelöst.

Gemäß der vorliegenden Erfindung wird erstmals eine perorale Applikationsform für Peptidarzneimittel vorgeschlagen, die in der Praxis herstellbar und anwendbar ist. Ein Vorteil besteht darin, daß das erfindungsgemäße Freigabesystem sowohl für schnelle Freisetzung als auch für retardierte Freisetzung oder eine Kombination aus schneller Freisetzung und retardierter Freisetzung geeignet ist. Weiterhin wird erst durch die vorliegende Erfindung die bekannt niedrige Resorptionsquote Peptidarzneistoffen, insbesondere Insulin, im GIT signifikant gesteigert.

Insbesondere stellt die vorliegende Erfindung eine perorale Applikationsform für Peptidarzneimittel zur Verfügung, enthaltend mindestens einen Peptidarzneistoff in einer Matrix, welche neben pharmazeutisch üblichen Träger- und Hilfsstoffen wenigstens ein hydrophiles Makromolekül enthält, ausgewählt aus der Gruppe bestehend aus: Gelatine, fraktionierter Gelatine, Kollagenhydrolysate, Gelatinederivate; sowie deren Mischungen.

Ferner stellt die vorliegende Erfindung unter anderem ein Verfahren zur Herstellung einer peroralen Applikationsform für Peptidarzneimittel zur Verfügung, wobei man mit wenigstens einem hydrophilen Makromolekül, ausgewählt aus der Gruppe bestehend aus: Gelatine, fraktionierte Gelatine, Kollagenhydrolysate, Gelatinederivate; sowie deren Mischungen, und dem Peptidarzneimittel eine pulverförmige Makromolekül-Arzneistoff-Mischung herstellt und die Mischung komprimiert.

Darüber hinaus stellt die vorliegende Erfindung ein Verfahren zur Herstellung einer sich langsam auflösenden peroralen Applikationsform für Peptidarzneimittel, dadurch gekennzeichnet, daß man
a) ein hydrophiles Makromolekül, ausgewählt aus der Gruppe bestehend aus: Gelatine, fraktionierte Gelatine, Gelatinederivate; sowie deren Mischungen, mit einem Maximum der Molekulargewichtsverteilung im Bereich von etwa 9,5 x 10⁴ - 10⁶ D auswählt,
b) das hydrophile Makromolekül bei einer Temperatur unterhalb der Inaktivierungstemperatur des Peptids mit Wasser in die Solform überführt,
c) den pH-Wert des Sols auf einen Wert zwischen dem des IEP's des hydrophilen Makromoleküls und dem des Peptids einstellt,
d) das Peptid in gelöster oder ungelöster Form dem Makromolekülsol zusetzt,
e) das Wasser entfernt,
f) das erhaltene Pulver nach üblichen Verfahren zu der Applikationsform preßt.

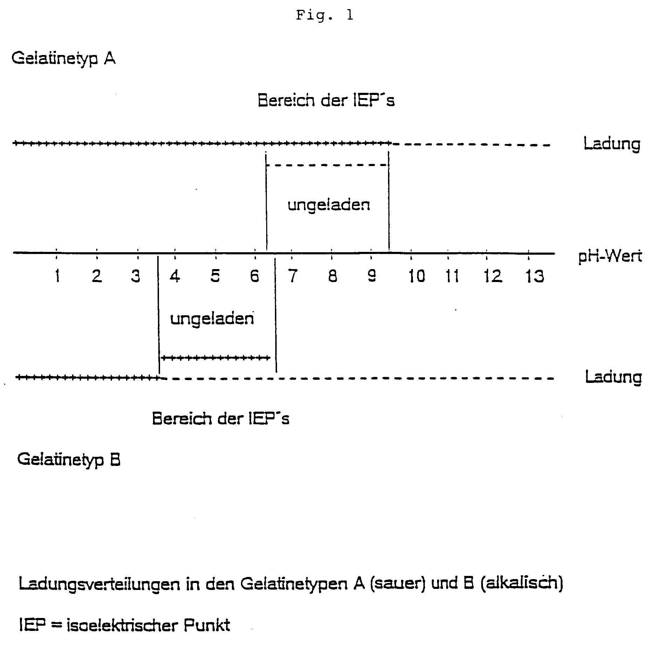

Fig. 1 zeigt eine schematische Darstellung der einstellbaren Ladungszustände von Gelatine in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

Die Inhalte der beiden internationalen (PCT)-Patentanmeldungen mit den Titeln "Pharmazeutisch applizierbares Nanosol sowie Verfahren zu seiner Herstellung" (81AL2730, entsprechend der deutschen Patentannnmeldung P 41 40 195.6) sowie "Sol-gesteuerte Thermokolloidmatrix auf Gelatinebasis für perorale Retardformen" (81AL2739, entsprechend der deutschen Patentanmeldung P 41 40 192.1) desselben Anmelders vom selben Tage werden auch zum Inhalt der vorliegenden Patentanmeldung gemacht.

Weitere Patentanmeldungen der ALFATEC-Pharma GmbH, gegebenenfalls auch der PAZ Arzneimittelentwicklungsgesellschaft mbH, von demselben Tage betreffen die Akutform von 2-Arylpropionsäurederivaten (81AL2731 = deutsche Patentanmeldung P 41 40 185.9), die Retardform von Dihydropyridinderivaten (81AL2732 = deutsche Patentanmeldung P 41 40 194.8), die Akutform von S- und R-Ibuprofen (81AL2733 0 deutsche Patentanmeldung P 41 40 179.4), die Retardform von S- und R-Ibuprofen (81AL2734 = deutsche Patentanmeldung P 41 40 172.7), die Akutform von S- und R-Flurbiprofen (81AL2735 = deutsche Patentanmeldung P 41 40 184.0), die Retardform von S- und R-Flurbiprofen (81AL2736 0 deutsche Patentanmeldung P 41 40 183.2) und die Retardform von Indolylessigsäurederivaten (81AL2737 = deutsche Patentanmeldung P 41 40 191.3). Ihre Offenbarung wird auch zum Gegenstand der Offenbarung der vorliegenden Patentanmeldung gemacht.

In der ersten der genannten internationalen (PCT)-Patentanmeldungen wird die Herstellung von kolloid-dispersen Systemen (Nanosolen) mit Gelatine beschrieben, in der letzteren (81AL2737) die Herstellung von retardiert und konstant (0. Ordnung) Wirkstoff freisetzenden, peroralen Arzneiformen auf Gelatinebasis. Peptidarzneistoffe, insbesondere Insulin, können gemäß der in den genannten Patentanmeldungen beschriebenen Verfahren in eine perorale Applikationsform gebracht werden. Als besonders vorteilhaft kann jedoch grundsätzlich die Kombination dieser Applikationsformen gesehen werden.

Insulin ist ein Peptidarzneistoff, der aus 51 Aminosäuren besteht, die in zwei Ketten (A- und B-Kette) angeordnet sind. Insulin ist bezüglich äußeren Einflüssen sehr empfindlich. So ist Hitze- und Alkaliempfindlichkeit, Empfindlichkeit gegenüber oxidierenden und reduzierenden Agenzien, sowie stark sauer reagierenden Substanzen bekannt. Aufgrund seines isoelektrischen Punktes (IEP) von 5,3 - 5,4 ist Insulin jedoch im schwach sauren Milieu bei pH 3-4, sowie im schwach alkalischen Milieu bei pH 7-8 ausreichend löslich und auch hinreichend stabil. In den angegebenen pH-Bereichen ist das Molekül jedoch positiv (pH kleiner IEP) bzw. negativ (pH größer IEP) geladen.

In einer besonderen Ausführungsform der vorliegenden Erfindung, die in Unteransprüchen beansprucht ist, liegen Peptidarzneistoffe, insbesondere Insulin, in einer Form vor, bei der der Peptidarzneistoff in geladener und gleichzeitig gelöster Form durch adsorptive Ladungskompensation (Pseudokoazervat) mit einer entgegengesetzt geladenen Gelatine oder einem Gelatinederivat assoziiert ist.

Im sauren Bereich unterhalb pH 5,3 - 5,4, wo das Insulinmolekül positiv geladen ist, kommt dafür nur negativ geladene Gelatine in Frage. Außer Gelatine Typ B lassen sich auch bestimmte Molfraktionen dieser Gelatine, sogenannte fraktionierte Gelatine, sowie Gelatinederivate, insbesondere succinylierte Gelatine, verwenden. Diese zeigen im angegebenen pH-Bereich gleiches Verhalten wie Gelatine Typ B. Als einziger ist der Typ B geeignet, der einen IEP kleiner 5,3 - 5,4 besitzen muß und damit bei pH-Werten oberhalb seines IEP negativ geladen ist. Umgekehrt ist Insulin bei pH-Werten größer 5,3 - 5,4 negativ geladen. Diese negative Ladung kann analog nur durch Gelatine Typ A, die bei pH-Werten größer 5,3 - 5,4 bis hin zu pH ca. 9,5 positiv geladen ist., kompensiert werden.

Bei einer nach diesem Prinzip hergestellten Arzneiform ist besonders der Gelatinetyp B zu bevorzugen. Es hat sich nämlich erstaunlicherweise folgendes gezeigt:

Nach Erreichen des Dickdarms bzw. Colons, wo physiologische pH-Werte von ca. 6-7,5 vorherrschen und die Insulinfreisetzung aus der Arzneiform beginnt, schützen die umhüllenden Gelatinepartikel das Insulinmolekül wirksam vor enzymatischem Abbau durch Peptidasen. Dabei macht sich noch ein zusätzlicher Effekt der Gelatine vorteilhaft bemerkbar. Die hochmolekularen Anteile der Gelatine (bevorzugt ab einem Molekulargewicht von ca. 10⁷ D) bilden sphärisch geformte Netzwerke aus. Durch diese Netzwerke ist ein Diffundieren der abbauenden Enzyme noch zusätzlich erschwert, sodaß das Insulinmolekül noch besser geschützt ist. Andererseits zeigen diese Gelatinepartikel bzw. Netzwerke eine gute Anhaftung an Schleimhautoberflächen, was optimale Voraussetzungen für die Resorption sicherstellt. Durch die pH-Verschiebung zu pH-Werten größer als 6 liegt nun das Insulin nicht mehr positiv geladen vor, sondern wird umgeladen und kann somit aus dem "Komplex" (Pseudokoazervat) mit der Gelatine, deren Ladung sich immer mehr in den negativen Bereich verschiebt, entlassen werden. Dieser "Umladungsvorgang" kann zusätzlich beschleunigt werden, indem erfindungsgemäß Puffersubstanzen (z.B. Dinatriumhydrogenphosphat) in der Gelatinematrix vorliegen, deren Pufferkapazitätsmaximum bei pH-Werten größer als 6 zu liegen kommt. Es ist aber zu betonen, daß es sich hierbei nicht um einen echten Einschlußkomplex, wie etwa bei Cyclodextrinen, handelt. Die Insulinfreisetzung erfolgt in jedem Falle ohne das bei Cyclodextrin-Verbindungen beispielsweise übliche vorgelagerte Gleichgewicht. Damit werden optimale Voraussetzungen für die Insulinresorption im Gastrointestinaltrakt geschaffen.

Um dieses Prinzip für eine perorale Arzneiform von Insulin oder auch anderen Arzneistoffen noch effektiver auszunutzen, kann die in der vorliegenden Erfindung beschriebene Arzneiform bevorzugt eine Zweischichttablette, oder noch besser Manteltablette darstellen. Die Tablette ist mit geeigneten Filmüberzügen, z.B. Eudragiten^{R} (Röhm-Pharma, Deutschland) magensaftresistent überzogen. Besonders bewährt haben sich Eudragit S, Mischungen aus Eudragit S und Eudragit RS-Typen, oder Mischungen aus Eudragit S, Eudragit L und Eudragit RS-Typen. Diese Filmüberzüge haben den Vorteil, daß sie bis zur Auflösung wasserundurchlässig sind und sich erst bei pH-Werten ab ca. 7 aufzulösen beginnen, also nachdem die Arzneiform sich bereits in unteren Darmabschnitten befindet oder bereits im Colon. Bis zu diesem Zeitpunkt ist damit die Arzneiform und der enthaltene Wirkstoff (Insulin) zusätzlich wirksam vor dem enzymatischen Abbau durch die Enzyme der Verdauungsflüssigkeit geschützt.

Die erste Schicht bzw. der Mantel der besagten Arzneiform ist nun so aufgebaut, daß eine relativ langsame (retardierte) Wirkstofffreigabe innerhalb von ca. 4 h erfolgt. Die zweite Schicht dagegen bzw. der Kern der Manteltablette ist so aufgebaut, daß eine schnelle (nicht retardierte) Wirkstofffreigabe erfolgt. Diese Kombination aus Akut- und Retardform in einer einzigen Tablette hat den Vorteil, daß die schnelle Insulinfreisetzung auf jeden Fall erst nach Erreichen des Colons stattfindet, wo bekanntlich nur noch ein peptidasearmes Medium anzutreffen ist.

Damit ist stets eine kontinuierliche Versorgung des Organismus mit Insulin gegeben, sodaß sich eine Anpassung an den Insulinbedarf eines Patienten nach Nahrungsaufnahme leicht vornehmen läßt. Auf diese Weise ist erfindungsgemäß eine Unabhängigkeit von der Insulininjektion zu erzielen und die Patienten-Compliance läßt sich entscheidend erhöhen.

Außer Insulin, worunter reguläres Insulin, mit Zink komplexiertes Insulin oder auch Globin-Zink-Insulin verstanden wird, sind für die vorliegende Erfindung auch andere Peptidarzneistoffe, die im Gastrointestinaltrakt enzymatisch inaktiviert werden können, geeignet, wie Octreocid, Desmopressin, Vasopressin, Triptorelin, körpereigene Peptidhormone wie Gonadotropin Releasing Hormon, Somatotropin Releasing Hormon, Corticotropin Releasing Hormon oder Thyreotropin Releasing Hormon, Polypeptidantibiotika, Ciclosporin, Buserelin, Calcitonin, Gonadorelin, Lysoprenin, Oxytocin, Protirelin, Hirudin, Glucagon, Enkephalin oder adrenocorticotropes Hormon. Stoffe zur Behandlung von AIDS (Renin-Antagonisten), Behandlung der Hypertonie (Renin-Antagonisten, Enalapril, Captopril), Antibiotika, die sich von Aminosäuren ableiten, Penicilline (Ampicillin), Cephalosporine (Cefalexin), Carbapeneme (Thienamycin), Interferone (alpha-Interferon), Impfstoffe.

Die vorliegende Erfindung schlägt außerdem ein einfaches Verfahren zur Herstellung der beschriebenen Arzneiformen vor.

Man wählt entsprechend der internationalen (PCT-)Anmeldung 81AL2739 zunächst eine höherviskose Gelatine mit entsprechender Bloomzahl, mit einem Maximum der Molekulargewichtsverteilung im Bereich 9,5 x 10⁴ - 10⁶, bevorzugt Typ B mit einem IEP im Bereich von 3,5 bis ca. 5,3 aus, die völlig von Fremdionen befreit ist. Die Gelatine, die für die retardierende Schicht bzw. den Mantel der erfindungsgemäßen Arzneiform verwendet werden kann, wird zunächst bei einer Temperatur, die oberhalb von 37°C liegt und unterhalb der Temperatur, bei der das Insulin bereits "inaktiviert" wird, mit Wasser in die Solform überführt. Die Gelatinekonzentrationen betragen üblicherweise 0,1 - 20 % (Gewichtsprozente), bevorzugt jedoch 0,1 - 5 %. Der pH des Sols wird durch Säure- bzw. Basenzusatz auf einen Wert eingestellt, der oberhalb des IEP's der verwendeten Gelatine und unterhalb des IEP's des eingesetzten Insulins liegt. Dadurch wird auf den Gelatinemolekülen ausreichende negative Ladung erzeugt, um die adsorptive Ladungskompensation (Pseudokoazervat) mit den Insulinmolekülen zu bewirken. Üblicherweise kann das Insulin, z.B. 50 - 500 I.E., direkt zu dem Gelatinesol gegeben werden und darin unter Rühren gelöst werden oder dem Gelatinesol bereits in gelöster Form zugesetzt werden. Die fortschreitende Ladungskompensation (Pseudokoazervatbildung) kann dabei beispielsweise durch eine einfache Leitfähigkeitsmessung des Systems verfolgt werden. Es kann erforderlich sein, den pH des Systems auf den vorgegebenen Wert nachzuregulieren, wenn dieser sich während des Herstellungsprozesses verschieben sollte.

Das Wasser kann nun durch bekannte Verfahren, wie z.B. Sprüh- oder Gefriertrocknung, entfernt werden, wobei der geforderte Zustand des Systems in trockener Form fixiert wird.

Völlig analog dazu ist ein zweites, trockenes System herzustellen, das die Grundlage für die zweite Schicht bzw. den Kern der erfindungsgemäßen Arzneiform bildet. Die dabei verwendete Gelatine vom gleichen Typ und mit identischem IEP besitzt bevorzugt ein Maximum der Molekulargewichtsverteilung unterhalb 10⁵, so daß eine nicht retardierte Freisetzung gewährleistet werden kann.

Die getrockneten Pulver können dann unter Zusatz üblicher pharmazeutischer Hilfsstoffe, wie beispielsweise Füllstoffe, Puffersubstanzen, Fließregulierungsmittel, Schmiermittel, Formentrennmittel auf geeigneten Tablettenpressen zu üblichen Tabletten oder zu Zweischicht- oder Manteltabletten verpreßt werden. Erstaunlicherweise zeichnen sich die erfindungsgemäßen Tabletten durch hohe Bruchfestigkeit und geringen Abrieb (Friabilität) aus.

Diejenige Schicht der Zweischichttablette, die nicht retardiert freisetzen soll, kann getrennt hergestellt und durch Überziehen mit einem der oben genannten Filmbildner vorisoliert werden.

Anschließend werden die erfindungsgemäß hergestellten üblichen Tabletten, Zweischicht- bzw. Manteltabletten nach üblichen Überzugsverfahren (beispielsweise in der Wirbelschicht, im Dragierkessel o.a.) mit den erwähnten Filmbildnern überzogen. Besonders vorteilhaft wird Eudragit S verwendet, oder Mischungen von Eudragit S mit Eudragit RS, z.B. im Mischungsverhältnis von 3:2.

Prinzipiell sind zur Herstellung der erfindungsgemäßen Applikationsform auch besonders die in der o.g. deutschen Patentanmeldung P 41 40 195.6 der ALFATEC-Pharma GmbH "Pharmazeutisch applizierbares Nanosol und Verfahren zu seiner Herstellung" genannten Vorgehensweisen und Verfahrensvarianten geeignet, die im folgenden noch einmal angeführt werden:

Es werden mehrere Verfahren zur Herstellung der Nanosole vorgeschlagen. Dabei handelt es sich um eine beispielhafte, unvollständige Aufzählung. Der Fachmann kann aufgrund seines Fachwissens selbstständig weitere Varianten im Rahmen der vorliegenden Erfindung ausarbeiten:

### Verfahren I

Dieses kann angewendet werden, wenn der Arzneistoff in einer Mischung aus:
einem mit Wasser mischbaren organischen Lösungsmittel und Wasser, oder
aus mehreren mit Wasser mischbaren organischen Lösungsmitteln und Wasser
löslich ist:
a) eine in den Vorversuchen ausgewählte Gelatine wird mit Wasser in Solform überführt;
b) der in den Vorversuchen gefundene pH-Wert der Lösung wird eingestellt;
c) ein oder mehrere mit Wasser mischbare(s), organische(s) Lösungsmittel, vorzugsweise Ethanol, Isopropanol oder Methanol, wird/werden zu dieser Lösung gegeben;
d) der Arzneistoff wird in fester Form zu der Lösung gegeben und gelöst;
e) das/die organische(n) Lösungsmittel wird/werden entfernt, vorzugsweise durch Eindampfen in Vakuum; dabei entsteht das Nanosol;
f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

### Verfahren II

Diese Ausführungsform kann angewendet werden, wenn der Arzneistoff eine Säure oder eine Base ist, deren Salz in Wasser löslich ist:
a) eine in den Vorversuchen ausgewählte Gelatine wird mit H₂O in die Solform überführt;
b) es wird ein solcher pH-Wert eingestellt, der die Salzbildung des Arzneistoffs ermöglicht;
c) der Arzneistoff wird unter Salzbildung in dem Gelatinesol gelöst;
d) durch Zugabe von Alkohol oder ähnlichen organischen Lösungsmitteln kann die Hydrathülle der Gelatinemoleküle gelockert werden;
e) durch Zugabe einer geeigneten Menge Säure oder Base wird der pH-Wert eingestellt, der zur Bildung des isoionischen Punkts (IIP) führt, dabei entsteht das Nanosol;
f) die kolloid-disperse Lösung wird wie in Verfahren I getrocknet.

Stufe d) ist fakultativ, jedoch bevorzugt.

### Verfahren III

Diese Ausführungsform kann angewendet werden, wenn der Arzneistoff ein Neutralstoff ist:
a) es wird ein Gelatinesol hergestellt, wie unter (1) a) und b) beschrieben.
b) eine zweite Lösung aus einem mit Wasser mischbaren organischen Lösungsmittel, vorzugsweise Ethanol, Methanol, Isopropanol, Aceton und dem Arzneistoff wird hergestellt.
c) die beiden Lösungen werden vereinigt.
d) das organische Lösungsmittel wird entfernt und die kolloid-disperse Lösung wird getrocknet.

### Verfahren IV

a) Wie unter (I) a) und b) beschrieben.
b) In einer zweiten Lösung wird ein kolloid-disperses System mit dem Arzneistoff kurzzeitig gebildet, jedoch ohne Gelatine.
c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind. Im Falle der Akutform für 2-Arylpropionsäurederivate seien als bevorzugt geeignete Verfahren die Varianten Nr. II und III genannt.

Für die erfindungsgemäßen Arzneimittel eignen sich alle Gelatinen, Gelatinederivate, Kollagenhydrolysate und fraktionierte Gelatine, sowie deren Mischungen Gelatinesorten, die einen erfindungsgemäß beschriebenen isoelektrischen Punkt (IEP) aufweisen, der nicht handelsüblich ist, können nach den Beispielen I bis III aus o.g. deutscher Patentanmeldung hergestellt werden.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

### Beispiele für die Herstellung von erfindungsgemäß besonders geeigneten Gelatinesorten mit isoelektrischen Punkten von 3,5 bis 9,5

### Beispiel I:

### Verfahren zur Erzielung eines IEP's von 7,5 bis 9,5

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

### Beispiel II:

### Verfahren zur Erzielung eines IEP's von 4 bis 7,5

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei 1,01 x 10⁵ bis 2,02 x 10⁵ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei 1,01 x 10⁵ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von 1,01 x 10⁵ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

### Beispiel III:

### Verfahren zur Erzielung eines IEP's von 3,5 bis 6

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung (M = 10⁴ - 10⁷ D) erhalten.

Niedrigmolekulare bis kaltwasserlösliche Gelatinesorten kann man durch thermischen Abbau bzw. enzymatisch erhalten.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

**Tabelle 1**

| Molekulargewichtsverteilung von verschiedenen bekannten Gelatinetypen bzw. von bekannten Kollagenhydrolysaten | | | | | | | |
|---|---|---|---|---|---|---|---|
| Molekulargewichtsverteilung (KD) | Natives Kollagen % | Gelatine Typ B % | Gelatine Typ A % | Kollagenhydrolysat Gelita®-Collagel A | Kollagenhydrolysat Gelita®-Collagel B | Kollagenhydrolysat Gelita®-Sol C | Elastinhydrolysat Gelita®-Gelastin |
| >360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5,2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| <1 | 0 | 0 | 0 | 6,5 | 7,0 | 34,0 | 0 |
| MG | 360 | 165 | 185 | 12-18 | 12-18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Übliche pharmazeutische Hilfsstoffe und/oder weitere Makromoleküle können, sofern sie technologisch erforderlich sind, in flüssigem oder getrocknetem Zustand den erfindungsgemäßen Nanosolen zugesetzt werden.

Zum Beispiel kann ein Zusatz von Polyvinylpyrrolidon im Mengenverhältnis Gelatine zu Polyvinylpyrrolidon im Bereich von 5:1 bis 500:1 geeignet sein.

Eine Akutform im Sinne der Erfindung, die z.B. zu Tabletten verarbeitet wird oder lyophilisiert werden soll, kann durch Zusatz von niedrigmolekularen Polyvinylpyrrolidonsorten im Bereich von 10:1 bis 50:1 in den technologischen Verarbeitungseigenschaften verbessert werden, ohne daß die Stabilität der Nanosole negativ beeinflußt wird.

Die in den folgenden Beispielen bevorzugten Herstellungsverfahren, Vorgehensweisen und Bezeichnungen beziehen sich wie folgt auf die deutschen Patentanmeldungen "Pharmazeutisch applizierbares Nanosol und Verfahren zu seiner Herstellung" (P 41 40 195.6) bzw. die oben genannten Verfahren und Beispiele:
Nanosol-Herstellung: Verfahren II und III
Gelatineherstellung: Beispiel I bis III
Vortest: siehe folgende Beschreibung:
Vortest:

Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und NH₂-Gruppen und dem pH-Wert der Lösung ab. Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen: Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, welche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-Wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken. Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den pKₛ-Wert der vorhandenen COOH- bzw. NH₃⁺ - Gruppen gegeben ist).

Die beiden beschriebenen Systeme für die retardierte und nicht retardierte Insulinfreisetzung lassen sich durch geeignete Granulationsmethoden auch zu Granulaten bzw. klassischen Pellets formen, Solche Granulate bzw. Pellets können beispielsweise in Hartgelatinekapseln abgefüllt werden. Granulate, Pellets und Hartgelatinekapseln sind üblicherweise mit den gleichen Filmbildnern, wie für die erfindungsgemäße Tablette angegeben, überzogen, um mindestens eine Magensaftresistenz zu erreichen. Auf diese Weise lassen sich beispielsweise Mischungen von schnell und verzögert freisetzenden Pellets in einer einzigen Arzneiform (Hartgelatinekapsel) realisieren, wobei die Pelletsorten zusätzlich mit verschiedenen Filmbildnern überzogen sein können. Damit wird es möglich, die Anpassung an den Insulinbedarf des Organismus noch genauer vorzunehmen, als dies mit einer Tablette ohnehin schon möglich ist.

Perorale Pelletarzneiformen zeichnen sich weiterhin dadurch aus, daß sie in ihren gastrointestinalen Transitzeiten wesentlich unabhängiger von physiologischen Einflußfaktoren, wie z.B. Art und Menge aufgenommener Nahrung u.a. sind, als single-unit Arzneiformen wie z.B. Tabletten.

Die in der vorliegenden Patentanmeldung beschriebenen peroralen Arzneiformen lassen sich auch vorteilhaft für andere Applikationswege einsetzen.

So kann eine erfindungsgemäße Tablette, insbesondere eine einfache Retardzubereitung zur Applikation von Peptidarzneistoffen in der Mundhöhle (bukkal oder sublingual) verwendet werden. Die bioadhäsiven Eigenschaften der Gelatine bewirken dabei ein Anheften an der Mundschleimhaut nach Kontakt mit physiologischer Flüssigkeit.

Erfindungsgemäß sprüh- oder gefriergetrocknete Pulver lassen sich vorteilhaft zur Entwicklung von Nasensprays oder Nasengelen (nasale Applikation) einsetzen. Nach Einstäuben in die Nasenhöhle haften die Gelatine/Arzneistoff-Partikel aufgrund bioadhäsiver Eigenschaften an der Nasenschleimhaut und zeigen eine Verweildauer von durchschnittlich 3 bis 4 Stunden in der Nase.

Um die physiologischen Hintergründe der Resorption von Arzneistoffen im allgemeinen und die verbesserte Resorptionsquote der erfindungsgemäßen Nanosole bzw. Pseudokoazervate ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:
a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basische Arzneistoffe bevorzugt im Darm resorbiert.

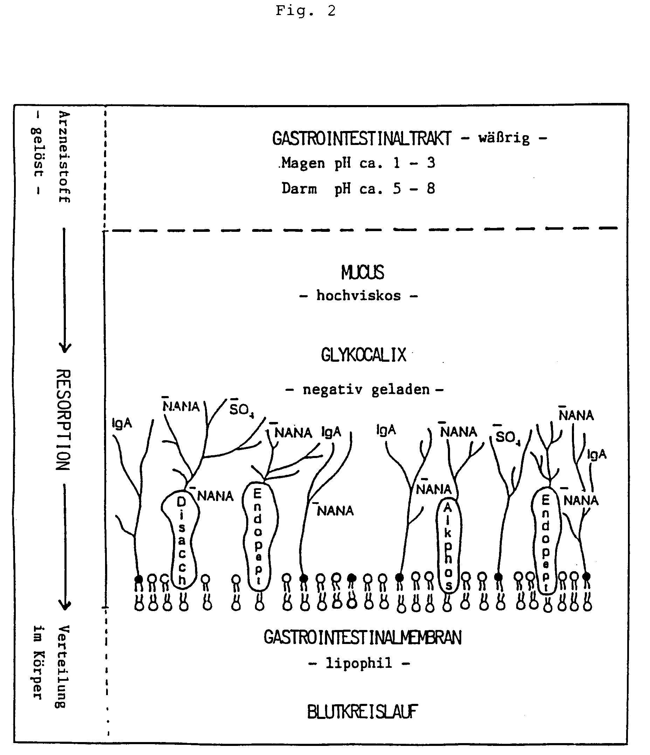

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Fig. 2), nämlich
- durch die Mucus-Schicht und eine wässerige, daran adhärierende Schicht
- die Zellmembranen der intestinalen Epithelzellen mit der daran kovalent gebundenen Glykocalix sowie
- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminsäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen.

Da die erfindungsgemäßen Wirkstoff-Nanosole bzw. Pseudokoazervate die Glykocalix ungehindert passieren können, ohne durch elektrostatische Effekte gebunden bzw. abgestoßen zu werden, erreichen sie damit auch die Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Folgende Beispiele sollen die Erfindung näher erläutern, ohne jedoch einen Anspruch auf Vollständigkeit zu erheben:

### Beispiel 1:

- Wirkstoff:: Normal-Insulin (DAB 9)
- Gelatine:: Typ B, völlig von Fremdionen befreit.
- IEP:: 3,5
- Bloomkennzahl:: 280 für retardierte Freisetzung 30 für schnelle Freisetzung
Jeweils 500 g der oben spezifizierten Gelatinen werden mit destilliertem Wasser bei 40° in die Solform überführt, so daß sich eine 5%ige Lösung ergibt. In jedem Sol wird ein pH-Wert von 3,9 eingestellt. Danach werden in beiden Ansätzen je 30000 I.E. Insulin der bezeichneten Spezifikation gelöst. Die erfolgende, adsorptive Ladungskompensation (Pseudokoazervatbildung) wird durch Leitfähigkeitsprüfung (z.B. mit einem mikroprozessorgesteuerten Hochleistungs-Konduktometer der Fa. WTW) verfolgt, bis keine Änderung der Gesamtleitfähigkeit mehr auftritt.

Anschließend werden beide Lösungen durch getrennte Sprühtrocknung bei einer Auslaßtemperatur des Sprühstroms von ca. 45 - 50°C in die trockene Form überführt.

Unter Zumischen üblicher pharmazeutischer Hilfsstoffe werden auf einer Tablettenpresse Manteltabletten, die als Kern das Insulin in nicht retardierter Form besitzen, hergestellt. Die Tablettenrohlinge werden dann im Dragierkessel durch Aufsprühen einer Lösung von Eudragit S und Eudragit RS im Verhältnis 3:2 in Aceton überzogen.

### Beispiel 2:

Analog Beispiel 1 werden zunächst die getrockneten Pulver hergestellt. Unter Zumischen üblicher pharmazeutischer Hilfsstoffe wird jedes Pulver für sich granuliert und zu Pellets geformt.
Anschließend werden die Pellets für schnelle Insulinfreisetzuung im Dragierkessel durch Aufsprühen einer acetonischen Lösung von Eudragit S und Eudragit RS im Verhältnis 3:2 überzogen, die Pellets für retardierte Insulinfreisetzung analog mit Eudragit S.

Beide Pelletsorten werden im Mischungsverhältnis 1:1 in Hartgelatinekapseln abgefüllt, die nach dem Verschließen mit Eudragit S überzogen werden.

### Beispiel 3:

- Wirkstoff:: Corticotropin, IEP im schwach alkalischen Bereich bei ca. 8
- Gelatine:: Typ A, völlig von Fremdionen befreit.
- IEP:: 9,0
- Bloomkennzahl:: 320 für retardierte Freisetzung 30 für schnelle Freisetzung
Jeweils 300 g der oben spezifizierten Gelatinen werden mit destilliertem Wasser bei 40° in die Solform überführt, so daß sich eine 3%ige Lösung ergibt. Mittels Salzsäure (2%) wird in jedem Sol ein pH-Wert von 8,5 eingestellt. Danach werden in beiden Ansätzen je 200 mg Corticotropin der bezeichneten Spezifikation gelöst.

Anschließend werden beide Lösungen durch getrennte Sprühtrocknung bei einer Auslaßtemperatur des Sprühstroms von ca. 45 - 50°C in die trockene Form überführt.

Unter Zumischen üblicher pharmazeutischer Hilfsstoffe werden auf einer Tablettenpresse Manteltabletten, die als Kern das Corticotropin in nicht retardierter Form besitzen, hergestellt.
Die Tablettenrohlinge werden dann im Dragierkessel durch Aufsprühen einer Lösung von Eudragit S in Aceton überzogen.

## Patentansprüche

1. Perorale Applikationsform für Peptidarzneimittel, enthaltend mindestens einen Peptidarzneistoff in einer sich unter physiologischen Bedingungen auflösenden Matrix aus Gelatine, fraktionierter Gelatine, Kollagenhydrolysat oder einem Gelatinederivat neben pharmazeutisch üblichen Trägern und Hilfsstoffen, wobei der(die) kolloidal oder gelöst vorliegende(n) Peptidarzneistoff(e) eine Ladung besitzen und die Moleküle des Matrixbildners eine gegensinnige Ladung besitzen.

2. Perorale Applikationsform für Peptidarzneimittel nach Anspruch 1, gekennzeichnet durch ein Komprimat.

3. Perorale Applikationsform für Peptidarzneimittel nach Patentanspruch 1 und/oder 2, dadurch gekennzeichnet, daß der Peptidarzneistoff Insulin ist.

4. Perorale Applikationsform für Peptidarzneimittel nach einem der Ansprüche 1-3, dadurch gekennzeichnet, daß die Gelatine eine Molekulargewichtsverteilung hat, deren Maximum bei 10⁴ bis 10⁷ D liegt.

5. Perorale Applikationsform für Peptidarzneimittel nach einem der Ansprüche 1-4, dadurch gekennzeichnet, daß der Peptidarzneistoff überwiegend in Gelatine mikroverkapselt ist.

6. Perorale Applikationsform für Peptidarzneimittel nach einem der Ansprüche 1-4, dadurch gekennzeichnet, daß ein schichtförmiger Aufbau vorliegt.

7. Perorale Applikationsform für Peptidarzneimittel nach einem der Ansprüche 1-6, dadurch gekennzeichnet, daß sie mit einem synthetischen oder natürlichen Überzug versehen ist.

8. Perorale Applikationsform für Peptidarzneimittel nach Anspruch 7, dadurch gekennzeichnet, daß sie als Manteltablette ausgebildet ist.

9. Perorale Applikationsform für Peptidarzneimittel nach einem der Ansprüche 1-8, dadurch gekennzeichnet daß eine zeitgesteuerte (langsam auflösende) Form mit einer schnell auflösenden Form kombiniert ist.

10. Applikationsform nach Anspruch 9, dadurch gekennzeichnet, daß die von außen erste Schicht bzw. der Mantel eine Retardform enthält, während die zweite Schicht bzw. der Kern eine Akutform enthält.

11. Verfahren zur Herstellung einer peroralen Applikationsform für Peptidarzneimittel nach Anspruch 1, dadurch gekennzeichnet, daß man
a) eine Gelatine, fraktionierte Gelatine, Kollagenhydrolysat oder ein Gelatinederivat nach ihrem isoelektrischen Punkt (IEP) so auswählt, daß ihr IEP mit dem Ladungszustand der Arzneistoffpartikel so abgestimmt ist, daß die Gelatine oder ihr Derivat bei einem bestimmten pH-Wert mit dem ungelösten Arzneistoff zu Ladungsneutralität führt,
b) die Gelatine oder ihr Derivat in die wäßrige Solform überführt,
c) den pH-Wert in Abhängigkeit von dem IEP der Gelatine auf einen solchen Wert einstellt, daß die sich bildenden Nanopartikel des Arzneistoffes nahezu oder vollständig ladungsneutral stabilisiert werden, und
d) vor oder nach Stufe c) den Arzneistoff in dem wäßrigen Gelatinesol löst oder eine Lösung des Arzneistoffes mit dem wäßrigen Gelatinesol vereinigt.

12. Verfahren zur Herstellung einer sich langsam auflösenden peroralen Applikationsform für Peptidarzneimittel nach Anspruch 1, dadurch gekennzeichnet, daß man
a) eine Gelatine, fraktionierte Gelatine oder ihr Derivat mit einem Maximum der Molekulargewichtsverteilung im Bereich von 9,5 x 10⁴ - 10⁶ D auswählt, die von Fremdionen frei ist,
b) die Gelatine bei einer Temperatur oberhalb 37° C und unterhalb der Inaktivierungstemperatur des Peptids mit Wasser in die Solform überführt,
c) den pH-Wert des Sols auf einen Wert zwischen dem des IEP's der Gelatine und dem des Peptids einstellt,
d) das Peptid in gelöster oder ungelöster Form dem Gelatinesol zusetzt und gegebenenfalls in dem Gelatinesol löst,
e) das Wasser entfernt,
f) das erhaltene Pulver nach üblichen Verfahren zu der Applikationsform preßt und
g) gegebenenfalls den Preßling mit einem Filmbildner überzieht.

13. Verfahren zur Herstellung einer peroralen Applikationsform für Peptidarzneimittel nach Anspruch 1, dadurch gekennzeichnet, daß man mit einer Gelatine, fraktionierter Gelatine, einem Kollagenhydrolysat oder einem Gelatinederivat, die sich in physiologischem Medium unter physiologischen Bedingungen auflösen, eine pulverförmige Gelatine-Arzneistoff-Mischung herstellt und die Mischung komprimiert.

14. Verfahren nach Anspruch 12 zur Herstellung einer sich zeitgesteuert langsam und schnell auflösenden Applikationsform, dadurch gekennzeichnet, daß man die Stufen a) bis e) mit einer zweiten Gelatine, einem Gelatinederivat oder Kollagenhydrolysat für die schnell auflösende Applikationsform durchführt, die ein Maximum der Molekulargewichtsverteilung unterhalb von 10⁵ enthält, und in Stufe f) die beiden erhaltenen Pulver zu Zweischicht- oder Manteltabletten preßt.

15. Verfahren nach einem der Ansprüche 11-14, dadurch gekennzeichnet, daß man Gelatine vom Typ B oder A ladungsabhängig einsetzt.

16. Verfahren nach einem der Ansprüche 11-14, dadurch gekennzeichnet, daß man Gelatine mit einem Anteil Mikrogel größer 10 Gew.-% einsetzt.

## Claims

1. Oral administration form for peptide medicaments, containing at least one peptide pharmaceutical substance in a matrix of gelatin, fractionated gelatin, collagen hydrolysate or a gelatin derivative which dissolves under physiological conditions, in addition to pharmaceutically customary excipients and auxiliaries, the peptide pharmaceutical substance(s) present in colloidal or dissolved form possessing a charge and the molecules of the matrix-forming agent possessing an opposite charge.

2. Oral administration form for peptide medicaments according to Claim 1, characterized by a tablet.

3. Oral administration form for peptide medicaments according to Patent Claim 1 and/or 2, characterized in that the peptide pharmaceutical substance is insulin.

4. Oral administration form for peptide medicaments according to one of Claims 1-3, characterized in that the gelatin has a molecular weight distribution whose maximum is at 10⁴ to 10⁷ D.

5. Oral administration form for peptide medicaments according to one of Claims 1-4, characterized in that the peptide pharmaceutical substance is mainly micro-encapsulated in gelatin.

6. Oral administration form for peptide medicaments according to one of Claims 1-4, characterized in that a layer-type construction is present.

7. Oral administration form for peptide medicaments according to one of Claims 1-6, characterized in that it is provided with a synthetic or natural coating.

8. Oral administration form for peptide medicaments according to Claim 7, characterized in that it is constructed as a layered tablet.

9. Oral administration form for peptide medicaments according to one of Claims 1-8, characterized in that a time-controlled (slowly dissolving) form is combined with a rapidly dissolving form.

10. Administration form according to Claim 9, characterized in that from the outside the first layer or the coat contains a depot form, while the second layer or the core contains an immediate-effect form.

11. Process for the production of an oral administration form for peptide medicaments according to Claim 1, characterized in that
a) a gelatin, fractionated gelatin, collagen hydrolysate or a gelatin derivative is selected according to its isoelectric point (IEP) such that its IEP is matched with the charge state of the pharmaceutical substance particles such that the gelatin or its derivative leads to charge neutrality with the undissolved pharmaceutical substance at a specific pH,
b) the gelatin or its derivative is converted into the agueous sol form,
c) the pH is adjusted as a function of the IEP of the gelatin to such a value that the nanoparticles of the pharmaceutical substance forming are almost or completely stabilized in a neutrally charged manner, and
d) before or after stage c), the pharmaceutical substance is dissolved in the aqueous gelatin sol or a solution of the pharmaceutical substance is combined with the aqueous gelatin sol.

12. Process for the production of a slowly dissolving oral administration form for peptide medicaments according to Claim 1, characterized in that
a) a gelatin, fractionated gelatin or its derivative with a maximum in the molecular weight distribution in the range from 9.5 x 10⁴ - 10⁶ D is selected, which is free from foreign ions,
b) the gelatin is converted into the sol form with water at a temperature above 37°C and below the inactivation temperature of the peptide,
c) the pH of the sol is adjusted to a value between that of the IEP of the gelatin and that of the peptide,
d) the peptide is added in dissolved or undissolved form to the gelatin sol and optionally dissolved in the gelating sol,
e) the water is removed,
f) the powder obtained is pressed to give the administration form by customary processes and
g) optionally the shaped article is coated with a film-forming agent.

13. Process for the production of an oral administration form for peptide medicaments according to Claim 1, characterized in that a powdered gelatin/pharmaceutical substance mixture is prepared with a gelatin, fractionated gelatin, a collagen hydrolysate or a gelatin derivative, which dissolve in physiological medium under physiological conditions, and the mixture is compressed.

14. Process according to Claim 12 for the production of a time-controlled slowly and rapidly dissolving administration form, characterized in that the stages a) to e) are carried out with a second gelatin, a gelatin derivative or collagen hydrolysate for the rapidly dissolving administration form which contains a maximum in the molecular weight distribution below 10⁵, and in stage f) the two powders obtained are pressed to give two-layer or layered tablets,

15. Process according to one of Claims 11-14, characterized in that gelatin of type B or A is employed independently of charge.

16. Process according to one of Claims 11-14, characterized in that gelatin having a content of microgel of greater than 10% by weight is employed.

## Revendications

1. Forme d'administration perorale de médicaments peptidiques, contenant au moins une substance médicamenteuse peptidique dans une matrice, se dissolvant dans des conditions physiologiques, de gélatine, de gélatine fractionnée, d'hydrolysat de collagène ou d'un dérivé de gélatine, à côté de supports et de substances auxiliaires pharmaceutiques classiques, dans laquelle la substance ou les substances peptidiques médicamenteuses présentes à l'état colloïdal ou dissous possèdent une charge et les molécules de la substance formant la matrice possèdent une charge de sens contraire.

2. Forme d'administration perorale de médicaments peptidiques suivant la revendication 1, caractérisée par un comprimé.

3. Forme d'administration perorale de médicaments peptidiques suivant la revendication 1 et/ou la revendication 2, caractérisée en ce que la substance peptidique médicamenteuse est l'insuline.

4. Forme d'administration perorale pour médicaments peptidiques suivant l'une des revendications 1 à 3, caractérisée en ce que la gélatine a une répartition de poids moléculaire dont le maximum a une valeur de 10⁴ à 10⁷ D.

5. Forme d'administration perorale de médicaments peptidiques suivant l'une des revendications 1 à 4, caractérisée en ce que la substance peptidique médicamenteuse est microencapsulée principalement dans de la gélatine.

6. Forme d'administration perorale pour médicaments peptidiques suivant l'une des revendications 1 à 4, caractérisée en ce qu'elle présente une structure en couches.

7. Forme d'administration perorale pour médicaments peptidiques suivant l'une des revendications 1 à 6, caractérisée en ce qu'elle est pourvue d'un revêtement synthétique ou naturel.

8. Forme d'administration perorale pour médicaments peptidiques suivant la revendication 7, caractérisée en ce qu'elle est réalisée comme comprimé enrobé.

9. Forme d'administration perorale de médicaments peptidiques suivant l'une des revendications 1 à 8, caractérisée en ce qu'une forme à action retardée (libération lente) est associée à une forme à libération rapide.

10. Forme d'administration suivant la revendication 9, caractérisée en ce que la première couche comptée de l'extérieur ou enveloppe contient une forme retardée, tandis que la seconde couche ou noyau contient une forme à action rapide.

11. Procédé de production d'une forme d'administration perorale de médicaments peptidiques suivant la revendication 1, caractérisé en ce que :
a) on choisit une gélatine, une gélatine fractionnée, un hydrolysat de collagène ou un dérivé de gélatine d'après son point isoélectrique (PIE) de manière que son PIE soit en accord avec l'état de charge des particules de médicaments afin que la gélatine ou son dérivé, pour une valeur de pH déterminée, mène à la neutralité de charge avec le médicament non dissous,
b) on fait prendre à la gélatine ou à son dérivé la forme d'un sol aqueux,
c) on ajuste le pH en fonction du PIE de la gélatine à une valeur telle que les nanoparticules de substances médicamenteuses qui se forment soient stabilisées avec neutralité de charge quasi totale ou totale, et
d) avant ou après l'étape c), on dissout la substance médicamenteuse dans le sol aqueux de gélatine ou on réunit une solution de la substance médicamenteuse et le sol aqueux de gélatine.

12. Procédé de production d'une forme d'administration perorale de médicaments peptidiques à libération lente suivant la revendication 1, caractérisé en ce que :
a) on choisit une gélatine, une gélatine fractionnée ou son dérivé avec un maximum de répartition de poids moléculaire dans la plage de 9,5 x 10⁴ à 10⁶ D, qui ne contient pas d'ions étrangers,
b) on fait prendre la forme d'un sol aqueux à la gélatine à une température au-dessus de 37°C et au-dessous de la température d'inactivation du peptide,
c) on ajuste le pH du sol à une valeur comprise entre celle du point isoélectrique de la gélatine et celle du peptide,
d) on ajoute le peptide sous la forme dissoute ou non dissoute au sol de gélatine et on le dissout éventuellement dans le sol de gélatine,
e) on élimine l'eau,
f) on comprime la poudre obtenue, par des procédés classiques, en la forme d'administration, et
g) on revêt éventuellement le comprimé d'une substance filmogène.

13. Procédé de production d'une forme d'administration perorale d'un médicament peptidique suivant la revendication 1, caractérisé en ce qu'on prépare un mélange en poudre gélatine-substance médicamenteuse avec une gélatine, une gélatine fractionnée, un hydrolysat de collagène ou un dérivé de gélatine qui se dissolvent en milieu physiologique dans des conditions physiologiques et on comprime le mélange.

14. Procédé suivant la revendication 12, pour la production d'une forme d'administration à libération lente et à libération rapide, caractérisé en ce qu'on conduit les étapes a) à e) avec une seconde gélatine, un dérivé de gélatine ou un hydrolysat de collagène pour la forme d'administration à libération rapide qui contient un maximum de répartition de poids moléculaire en dessous de 10⁵ et dans l'étape f), on comprime les deux poudres obtenues en des comprimés à deux couches ou à enveloppe.

15. Procédé suivant l'une des revendications 11 à 14, caractérisé en ce qu'on utilise de la gélatine de type B ou de type A en fonction de la charge.

16. Procédé suivant l'une des revendications 11 à 14, caractérisé en ce qu'on utilise de la gélatine contenant une proportion de microgel supérieure à 10 % en poids.
